# EUROPEAN PATENT SPECIFICATION

(11) **EP 3 864 004 B1**
(45) Date of publication and mention of the grant of the patent: **28.09.2022**
(21) Application number: 19795055.3
(22) Date of filing: 09.10.2019
(51) Int. Cl.: C07C 273/18, C07C 275/16

(54) **PROCESS FOR THE SYNTHESIS OF CARGLUMIC ACID**
VERFAHREN ZUR SYNTHESE VON CARGLUMSÄURE
PROCÉDÉ DE SYNTHÈSE D'ACIDE CARGLUMIQUE

(30) Priority: 11.10.2018 IT 201800009372
(43) Date of publication of application: 18.08.2021
(73) Proprietor: Procos S.p.A., 28062 Cameri (NO) (IT)
(72) Inventor: MORANA, Fabio, 28100 Novara (IT); GRANDE, Vincenzo, 28066 Galliate (NO) (IT); GOBBATO, Stefano, 28062 Cameri (NO) (IT); ROLETTO, Jacopo, 10127 Torino (IT); PAISSONI, Paolo, 10040 Druento (TO) (IT)
(74) Representative: Bianchetti & Minoja SRL
(86) International application number: PCT/IB2019/058593
(87) International publication number: WO 2020/075082

(56) References cited:
- CN-A- 107 033 035

## Description

The invention relates to a process for the synthesis of carglumic acid by addition reaction of sodium cyanate to L-glutamic acid in an aqueous medium in the presence of a compound containing lithium, and acidification.

### Background to the invention

Carglumic acid (N-carbamoyl-L-glutamic acid, CGA) is an analogue of N-acetylglutamate, a natural activator of carbamoyl phosphate synthetase, the enzyme involved in the first reaction of the urea cycle. Although carbamoyl phosphate synthetase has a lower affinity for carglumic acid than N-acetylglutamate, carglumic acid seems to stimulate the enzyme effectively, because it crosses the mitochondrial membrane of the hepatocyte (wherein this stage of the urea cycle takes place) more easily than N-acetylglutamate.

Urea cycle disorders are rare diseases which may occur at birth (total deficiency) or later (partial deficiency), and are due to a deficiency of one or more of the enzymes necessary for urea synthesis. Non-transformation of ammonium to urea is followed by an accumulation of ammonia in the plasma, and consequently in the tissues. Hyperammonaemia gives rise to encephalopathy, leading to neurological consequences and psychomotor retardation, or even the death of the infant; at later stages of life it can cause lethargy, behavioural disorders and, less frequently, encephalopathy and death. N-acetylglutamate synthetase deficiency is the rarest hereditary urea cycle abnormality. Patients suffering from said disorder are generally treated with sodium phenylbutyrate, which promotes the elimination of nitrogenous metabolites, bypassing the urea cycle. However, phenylbutyrate is only partly effective, and must be supported by strict dietary protein restrictions. Conversely, in the majority of patients treated with carglumic acid before the appearance of permanent brain lesions, growth and psychomotor development are normal. On an indirect comparison, carglumic acid proves more effective than sodium phenylbutyrate and does not require concomitant protein restrictions. Moreover, it has no serious adverse effects.

Carglumic acid can be synthesised from L-glutamic acid or the sodium salt thereof, both of which are commercially available at a low cost. The other reactive species is usually a cyanate, which is preferable to urea due to its higher reactivity, as a result of which it does not require drastic reaction conditions.

L-glutamic acid, the starting cyanate and carglumic acid are reactive species in a basic medium, usually giving rise to the formation of considerable by-products due to the orthogonal reactivity of the reagents or to the breakdown of the product, and significantly reduce both the yield and the quality of the product obtained.

In fact, none of the procedures described in the literature reports that carglumic acid was obtained with a purity level satisfactory for pharmaceutical use.

CN101168518 describes a process wherein glutamic acid, potassium cyanate and potassium hydroxide are reacted before acidifying with hydrochloric acid and hydantoic acid. Said process requires an additional crystallisation. Moreover, the use of hydantoic acid is not straightforward from the industrial standpoint, and traces thereof, or of its by-products, can easily be found in the end product.

CN107033035 describes a process that uses sodium glutamate and sodium cyanate, acidifying with sulfuric acid. The purities reported are unsatisfactory, and additional purifications are also required.

CN105601542 describes an improvement in the quality of the product compared with CN101168518 due to the use of a mixture of inorganic acids instead of hydrochloric acid alone. However, the purities obtained are only just over 90%.

CN101440042 describes the reaction between glutamic acid, ammonium formate and sodium hydroxide at 100°C. Said procedure is hazardous, because ammonium formate is a source of gaseous hydrogen; the drastic reaction conditions contribute to increasing the risks of the process. Moreover, the yields reported are modest, and there are no purity data.

CN101481336 describes the reaction between sodium glutamate and urea in the presence of microwaves, and is therefore only applicable on a laboratory scale for exploratory or analysis purposes.

In view of the factors described above, the need is still felt for a process able to limit or wholly eliminate the formation of certain impurities, thus enabling carglumic acid to be obtained with a purity sufficient for pharmaceutical use, and at the same time requiring a smaller number of operations than the known processes.

### Description of the invention

It has now been found that carglumic acid can be obtained with high yields and high purity by using the particular reactivity of its lithium salt with 1:1 stoichiometry (monolithium carglutamate or lithium monocarglutamate). Said salt is novel, and constitutes a further subject of the invention.

The process according to the invention produces carglumic acid with a purity exceeding 99.8% directly by filtration of the solid from the reaction mixture, with no need for further purifications.

The formation of carglumic acid lithium salt as intermediate drastically reduces or actually eliminates some degradation impurities of the end product which tend to form in the presence of bases containing sodium or potassium, and therefore notoriously make at least two further crystallisations necessary, thus increasing the processing time in industrial plants and leading to a significant increase in the costs of the process and the cost of the product obtained therefrom.

It has also surprisingly been found that the formation of the intermediate lithium salt drastically reduces the known tendency of L-glutamic acid to cyclise intramolecularly in basic aqueous solution to give L-pyroglutamic acid (scheme 1), one of the impurities often present in the final carglumic acid. As reported in the experimental examples, when the same reaction is conducted with lithium hydroxide, sodium hydroxide and potassium hydroxide, a tendency to inhibit said cyclisation is observed, which reduces the percentage of L-pyroglutamic acid about 10-fold, with a significant impact on both the yield and the quality of the solid carglumic acid, which is filtered directly from the reaction mixture; in fact, unlike the solid obtained when a lithium base is used, the solids obtained in the presence of sodium or potassium bases not only present considerably lower isolated yields, but also require further purification stages.

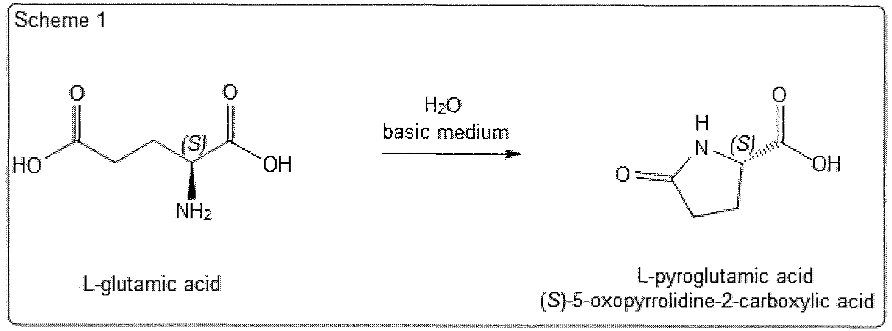

The process according to the invention comprises the addition reaction of sodium cyanate to L-glutamic acid in an aqueous medium in the presence of a compound containing lithium, optional concentration of the aqueous phase until precipitation of carglumic acid lithium salt with 1:1 stoichiometry, and acidification to obtain carglumic acid having an HPLC purity > 99.8%, with no need for further purifications.

Scheme 2 illustrates the two embodiments of the process according to the invention (with or without isolation of the lithium salt).

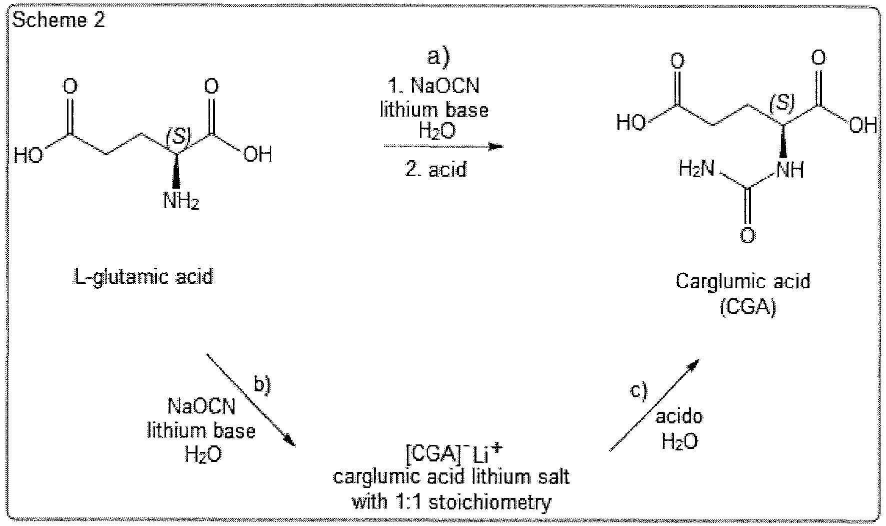

### Detailed description of the invention

The order of addition of the solvents, raw materials, acids or bases may differ from that reported below.

In one embodiment of the invention, the process is conducted as follows:
1 mole of L-glutamic acid is reacted with 0.8-2 moles of sodium cyanate, preferably 0.9-1.2 moles, in the presence of 0.6-1.5 moles, preferably 0.8-1.2 moles, of a compound containing lithium selected from lithium hydroxide monohydrate, anhydrous lithium hydroxide, metallic lithium, lithium carbonate, lithium hexamethyldisilazide, tribasic lithium phosphate, dibasic lithium phosphate, monobasic lithium phosphate, lithium bicarbonate, lithium sulfate, lithium hydrogen sulfate, lithium chloride, lithium acetate, lithium formate, lithium trifluoroacetate, lithium fluoride, lithium bromide, lithium iodide, lithium oxide, lithium tert-butoxide, straight or branched C1-C6 alkyllithium, lithium diisopropylamide (LDA) and lithium diethylamide, in 1-50 volumes of water, preferably 3-20 volumes, at a temperature ranging between 0 and 90°C, preferably between 20 and 60°C. When the disappearance of the L-glutamic acid has been monitored, an amount of an acid selected from aqueous HCl, nitric acid, concentrated sulfuric acid and aqueous sulfuric acid sufficient to reach a pH value between 0 and 3 is added. The carglumic acid thus filtered presents an HPLC purity > 99.8%, and therefore does not need any further purifications.

Alternatively, when the disappearance of the L-glutamic acid has been monitored, the aqueous phase is concentrated until the carglumic acid lithium salt precipitates with 1:1 stoichiometry, and the solid is filtered. An amount of an acid selected from aqueous HCl, concentrated sulfuric acid and aqueous sulfuric acid is added to the salt obtained, dissolved in 1-30 volumes of water, preferably 3-10 volumes, until a pH of between 0 and 3 is reached. The carglumic acid is filtered, and presents an HPLC purity > 99.8%, and therefore does not need any further purifications.

The invention is illustrated in detail in the examples below.

### Example 1 (Scheme 2 step a)

57.1 g (1.36 moles) of lithium hydroxide monohydrate is added to a reactor containing 1.2 L of water. After complete dissolution, 88.4 g (1.36 moles) of sodium cyanate is added, followed by 200 g (1.36 moles) of L-glutamic acid, and the mixture is stirred at 30°C for 24 h. 30% by weight of aqueous HCl is then added until a pH value below 3 is reached, at which point precipitation of the product is observed. The filtered solid is washed with water (3 x 30 mL) and dried under vacuum at 50°C for 24 h. 237 g of carglumic acid is obtained (yield 91.7%), with an HPLC purity of 99.86%.

The percentage of L-pyroglutamic acid in the reaction mixture after 24 h is 1.06%.

### Example 2 (Scheme 2 step b)

47.6 g (1.13 moles) of lithium hydroxide monohydrate is added to a reactor containing 1.0 L of water. After complete dissolution, 73.7 g (1.13 moles) of sodium cyanate is added, followed by 167 g (1.13 moles) of L-glutamic acid, and the mixture is stirred at 30°C for 24 h. The solution is then concentrated at low pressure until the precipitation of carglumic acid lithium salt with 1:1 stoichiometry, which is filtered and dried under vacuum at 50°C for 24 h. 200 g of intermediate is isolated (yield 90.4%).

¹H-NMR (D₂O, 400 MHz):
δ 4.00 - 3.92 (m, 1H), 2.30 - 2.18 (m, 2H), 2.09 - 1.97 (m, 1H), 1.91 - 1.78 (m, 1H).

¹³C-NMR (D₂O, 100 MHz):
δ 182.5, 180.1, 160.9, 55.6, 34.1, 29.1.

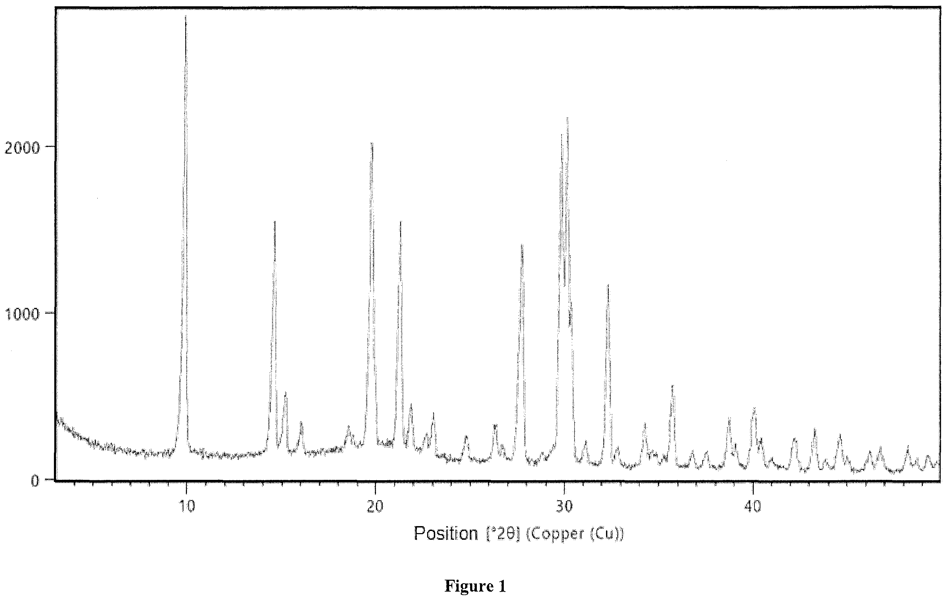

Figure 1 shows the X-ray powder diffractogram, having characteristic peaks at the diffraction angles 9.8°, 14.5°, 15.1°, 16.0°, 19.8°, 21.2°, 27.7°, 28.8°, 30.1°, 30.4°, 32.3°, 35.7°, 38.7° and 40.0° (2Θ ±0.1°, CuK).

### Example 3 (Scheme 2, step c)

200 g of carglumic acid lithium salt with 1:1 stoichiometry (1.02 moles) is dissolved in 1 L of water, and 30% by weight of aqueous HCl is added to the solution until a pH value below 3 is reached, at which point precipitation of the product is observed. The filtered solid is washed with water (3 x 20 mL) and dried under vacuum at 50°C for 24 h. 192 g of carglumic acid is obtained (yield 99.0%), with an HPLC purity of 99.88%.

### Example 4 (Scheme 2, step a)

50.3 g (0.68 moles) of lithium carbonate is added to a reactor containing 0.6 L of water. After complete dissolution, 44.2 g (0.68 moles) of sodium cyanate is added, followed by 100 g (0.68 moles) of L-glutamic acid, and the mixture is stirred at 30°C for 24 h. 30% by weight of aqueous HCl is then added until a pH value below 3 is reached, at which point precipitation of the product is observed. The filtered solid is washed with water (3 x 10 mL) and dried under vacuum at 50°C for 24 h. 113.6 g of carglumic acid is obtained (yield 88.0%), with an HPLC purity of 99.82%.

### Example 5 (Scheme 2, step a)

6.6 g (0.95 moles) of metallic lithium is added to a reactor containing 0.84 L of water. After complete dissolution, 61.9 g (0.95 moles) of sodium cyanate is added, followed by 140 g (0.95 moles) of L-glutamic acid, and the mixture is stirred at 30°C for 24 h. 30% by weight of aqueous HCl is then added until a pH value below 3 is reached, at which point precipitation of the product is observed. The filtered solid is washed with water (3 x 30 mL) and dried under vacuum at 50°C for 24 h. 162.6 g of carglumic acid is obtained (yield 90.1%), with an HPLC purity of 99.89%.

### Example 6 (Scheme 2, step a)

89.8 g (1.36 moles) of lithium acetate is added to a reactor containing 1.2 L of water. After complete dissolution, 88.4 g (1.36 moles) of sodium cyanate is added, followed by 200 g (1.36 moles) of L-glutamic acid, and the mixture is stirred at 30°C for 24 h. 30% by weight of aqueous HCl is then added until a pH value below 3 is reached, at which point precipitation of the product is observed. The filtered solid is washed with water (3 x 30 mL) and dried under vacuum at 50°C for 24 h. 226 g of carglumic acid is obtained (yield 87.5%), with an HPLC purity of 99.84%.

### Comparative example 7

54.4 g (1.36 moles) of sodium hydroxide is added to a reactor containing 1.2 L of water. After complete dissolution, 88.4 g (1.36 moles) of sodium cyanate is added, followed by 200 g (1.36 moles) of L-glutamic acid, and the mixture is stirred at 30°C for 24 h. 30% by weight of aqueous HCl is then added until a pH value below 3 is reached, at which point precipitation of the product is observed. The filtered solid is washed with water (3 x 30 mL) and dried under vacuum at 50°C for 24 h. 214.5 g of carglumic acid is obtained (yield 83.0%), with an HPLC purity of 97.3%. The percentage weight of the L-pyroglutamic acid in the reaction mixture after 24 h amounts to 5.06%.

### Comparative example 8

76.3 g (1.36 moles) of potassium hydroxide is added to a reactor containing 1.2 L of water. After complete dissolution, 88.4 g (1.36 moles) of sodium cyanate is added, followed by 200 g (1.36 moles) of L-glutamic acid. The mixture is stirred at 30°C for 24 h. 30% by weight of aqueous HCl is then added until a pH value below 3 is reached, at which point precipitation of the product is observed. The filtered solid is washed with water (3 x 30 mL) and dried under vacuum at 50°C for 24 h. 189.1 g of carglumic acid is obtained (yield 73.2%), with an HPLC purity of 96.7%. The percentage weight of the L-pyroglutamic acid in the reaction mixture after 24 h amounts to 10.40%.

## Claims

1. A process for the preparation of carglumic acid which comprises the addition reaction of sodium cyanate to L-glutamic acid in an aqueous medium in the presence of a lithium compound, optional concentration of the aqueous phase to precipitate the carglumic acid lithium salt in 1:1 stoichiometry and acidification to obtain carglumic acid.

2. The process according to claim 1 which comprises the addition reaction of sodium cyanate to L-glutamic acid in an aqueous medium in the presence of a lithium compound, and the acidification.

3. The process according to claim 1 which comprises the addition reaction of sodium cyanate to L-glutamic acid in an aqueous medium in the presence of a lithium compound, concentration of the aqueous phase to precipitate carglumic acid lithium salt in 1:1 stoichiometry and acidification.

4. A process according to any one of claims 1 to 3 wherein the lithium compound is selected from lithium hydroxide monohydrate, anhydrous lithium hydroxide, metal lithium, lithium carbonate, lithium hexamethyldisilazide, tribasic lithium phosphate, dibasic lithium phosphate, monobasic lithium phosphate, lithium bicarbonate, lithium sulfate, lithium hydrogen sulfate, lithium chloride, lithium acetate, lithium formate, lithium trifluoroacetate, lithium fluoride, lithium bromide, lithium iodide, lithium oxide, lithium tert-butoxide, straight or branched C1-C6 alkyllithium, lithium diisopropylamide and lithium diethylamide.

5. The process according to claim 4 wherein the lithium compound is lithium hydroxide monohydrate.

6. A process according to any one of claims 1 to 5 wherein the acidification is carried out by addition of aqueous HCl, nitric acid, concentrated sulphuric acid or aqueous sulphuric acid, at a pH below 3.

7. Carglumic acid lithium salt with 1:1 stoichiometry.

8. The carglumic acid lithium salt according to claim 7 having characteristic peaks at the diffraction angles 9.8°, 14.5°, 15.1°, 16.0°, 19.8°, 21.2°, 27.7°, 28.8°, 30.1°, 30.4°, 32.3° 35.7°, 38.7° and 40.0° (2Θ ± 0.1°, CuK).

## Patentansprüche

1. Verfahren zur Herstellung von Carglumsäure, das umfasst: die Additionsreaktion von Natriumcyanat an L-Glutaminsäure in einem wässrigen Medium in Gegenwart einer Lithiumverbindung, optional die Konzentration der wässrigen Phase zur Ausfällung des Lithiumsalzes der Carglumsäure in einer 1:1-Stöchiometrie und die Ansäuerung zum Erhalt von Carglumsäure.

2. Verfahren nach Anspruch 1, umfassend die Additionsreaktion von Natriumcyanat an L-Glutaminsäure in einem wässrigen Medium in Gegenwart einer Lithiumverbindung und die Ansäuerung.

3. Verfahren nach Anspruch 1, umfassend die Additionsreaktion von Natriumcyanat an L-Glutaminsäure in einem wässrigen Medium in Gegenwart einer Lithiumverbindung, die Konzentration der wässrigen Phase zur Ausfällung des Lithiumsalzes der Carglumsäure in einer 1:1-Stöchiometrie und die Ansäuerung.

4. Verfahren nach einem der Ansprüche 1 bis 3, wobei die Lithiumverbindung ausgewählt ist aus Lithiumhydroxid-Monohydrat, wasserfreiem Lithiumhydroxid, Metalllithium, Lithiumcarbonat, Lithiumhexamethyldisilazid, dreibasischem Lithiumphosphat, zweibasischem Lithiumphosphat, einbasischem Lithiumphosphat, Lithiumbicarbonat, Lithiumsulfat, Lithiumhydrogensulfat, Lithiumchlorid, Lithiumacetat, Lithiumformiat, Lithiumtrifluoracetat, Lithiumfluorid, Lithiumbromid, Lithiumiodid, Lithiumoxid, Lithium-tert.-butoxid, gerades oder verzweigtes C1-C6-Alkyllithium, Lithiumdiisopropylamid und Lithiumdiethylamid.

5. Verfahren nach Anspruch 4, wobei die Lithiumverbindung Lithiumhydroxid-Monohydrat ist.

6. Verfahren nach einem der Ansprüche 1 bis 5, wobei die Ansäuerung durch Zugabe von wässriger HCl, Salpetersäure, konzentrierter Schwefelsäure oder wässriger Schwefelsäure bei einem pH-Wert unter 3 durchgeführt wird.

7. Carglumsäure-Lithiumsalz mit 1:1-Stöchiometrie.

8. Carglumsäure-Lithiumsalz nach Anspruch 7 mit charakteristischen Peaks bei den Beugungswinkeln 9,8°, 14,5°, 15,1°, 16,0°, 19,8°, 21,2°, 27,7°, 28,8°, 30,1°, 30,4°, 32,3° 35,7°, 38,7° und 40,0° (2Θ ± 0,1°, CuK).

## Revendications

1. Procédé destiné à la préparation d'acide carglumique, qui comprend la réaction par addition de cyanate de sodium à de l'acide L-glutamique dans un milieu aqueux en présence d'un composé de lithium, la concentration facultative de la phase aqueuse dans le but de précipiter le sel de lithium de l'acide carglumique dans un rapport stœchiométrique de 1:1 et l'acidification afin d'obtenir l'acide carglumique.

2. Procédé selon la revendication 1, qui comprend la réaction par addition de cyanate de sodium à de l'acide L-glutamique dans un milieu aqueux en présence d'un composé de lithium et l'acidification.

3. Procédé selon la revendication 1, qui comprend la réaction par addition de cyanate de sodium à de l'acide L-glutamique dans un milieu aqueux en présence d'un composé de lithium, la concentration de la phase aqueuse dans le but de précipiter le sel de lithium de l'acide carglumique dans un rapport stœchiométrique de 1:1 et l'acidification.

4. Procédé selon l'une quelconque des revendications 1 à 3, dans lequel le composé de lithium est choisi parmi le monohydrate d'hydroxyde de lithium, l'hydroxyde de lithium anhydre, le lithium métallique, le carbonate de lithium, l'hexaméthyldisilazide de lithium, le phosphate de lithium tribasique, le phosphate de lithium dibasique, le phosphate de lithium monobasique, le bicarbonate de lithium, le sulfate de lithium, l'hydrogénosulfate de lithium, le chlorure de lithium, l'acétate de lithium, le formiate de lithium, le trifluoroacétate de lithium, le fluorure de lithium, le bromure de lithium, l'iodure de lithium, l'oxyde de lithium, le tert-butoxyde de lithium, l'alkyl(en C₁-C₆ à chaîne droite ou à chaîne ramifiée) de lithium, le diisopropylamidure de lithium et le diéthylamidure de lithium.

5. Procédé selon la revendication 4, dans lequel le composé de lithium est le monohydrate d'hydroxyde de lithium.

6. Procédé selon l'une quelconque des revendications 1 à 5, dans lequel l'acidification est mise en œuvre par addition de HCl aqueux, d'acide nitrique, d'acide sulfurique concentré ou d'acide sulfurique aqueux, à un pH qui est inférieur à 3.

7. Sel de lithium de l'acide carglumique avec un rapport stœchiométrique de 1:1.

8. Sel de lithium de l'acide carglumique selon la revendication 7, qui possède des pics caractéristiques aux angles de diffraction 9,8°, 14,5°, 15,1°, 16,0°, 19,8°, 21,2°, 27,7°, 28,8°, 30,1°, 30,4°, 32,3°, 35,7°, 38,7°, et 40,0° (2Θ ± 0,1 °, CuK).
